# EUROPEAN PATENT APPLICATION

(11) **EP 0 629 408 A1**
(43) Date of publication of application: **21.12.1994**
(21) Application number: 93401551.2
(22) Date of filing: 16.06.1993
(51) Int. Cl.: A61K 37/64, A61K 31/415, A61K 45/06

(54) **Combination of angiotensin converting enzyme inhibitors and AII antagonists**

(71) Applicant: LABORATOIRES MERCK, SHARP & DOHME-CHIBRET, F-75008 Paris (FR); INSTITUT NATIONAL DE LA SANTE ET DE LA RECHERCHE MEDICALE (INSERM), 75654 Paris Cédex 13 (FR)
(72) Inventor: Hannedouche, Thierry, F-67000 Strasbourg (FR); Schmitt, Francois, F-78700 CONFLANS STE HONORINE (FR); Lacour, Bernard, F-94240 L'Hay Les Roses (FR); Madonna, Olivier, F-92400 Courbevoie (FR)
(74) Representative: Martin, Jean-Jacques

(57) **Abstract**

Pharmaceutical composition to enhance renal blood flow comprising as active ingredient a combination of at least one ACE (Angiotensin Converting Enzyme) inhibitors and at least one AII receptor antagonist.

## Description

The present invention relates to a combination of ACE inhibitors and AII antagonists.

More particularly the present invention concerns a combined pharmaceutical composition comprising a combination of at least one ACE (Angiotensin Converting Enzyme) inhibitor and at least one AII antagonist (Angiotensin II antagonist) for simultaneous, separate or sequential administration.

According to the present invention, the angiotensin converting enzyme inhibitor can be in first dosage form and the AII antagonist is in a second dosage form, or preferably the angiotensin converting enzyme inhibitor and the AII antagonist can be in a single unit dosage.

The composition of the present invention exhibits an enhanced effect on renal blood and can be used in a method of increasing renal blood flow rate in human.

Renal selective effect is not present with the single entities alone. Angiotensin is produced by two pathways, one classical pathway and a non-classical pathway. ACE only blocks classical pathways, while the combination blocks both, producing a blockade thAT results in an enhanced renal dilation.

Accordingly, the pharmaceutical composition of the present invention can also be used for the treatment or prevention of cardiovascular diseases.

Particularly suitable as ACE inhibitors in the composition of the present invention the following drugs can be mentioned : enalapril, lisinopril, ceranapril, imidapril, captopril, DU-1777, zabicipril, utibapril, AB-47, cilazapril, zofenopril, fosinopril, delapril, spirapril, perindopril, libenzapril, moexipril, MDL-100240, quinapril, trandolapril, benazepril, quinaprilat, FPL-66564, Synecor, Prentyl, BIBS39, temocapril, idrapril, ramipril.

As ACE inhibitor Enalapril, Lisinopril, Captopril, Ramipril, Cilazapril and Quinapril are preferred.

Several non-peptide compounds have been described as A II antagonists suitable for the composition of the present invention. Illustrative of such compounds are those disclosed in U.S. Patents 4,207,324; 4,340,598; 4,576,958; and 4,582,847 in European Patent Applications 028,834; 245,637; 253,310; and 291,969; and in articles by A.T. Chiu, et al. (Eur. J. Pharm. Exp. Therap., 157, 13-21 (1988)) and by P.C. Wong, et al. (J; Pharm. Exp. Therap. 247, 1-7 (1988)). All of the U.S. Patents, European Patent Applications 028,834 and 253,310 and the two articles disclose substituted imidazole compounds which are generally bonded through a lower alkyl bridge to a substituted phenyl. European patent Application 245,637 discloses derivatives of 4, 5, 6, 7-tetrahydro-2H-imidazo[4, 5-c]-pyridine-6-carboxylic acid and analogs thereof as antihypertensive agents.

Substituted imidazoles have been disclosed in patents to DuPont (EPO 253,310 and EPO 324,377) focusing on the design of Angiotensin II Antagonists. Substituted benzimidazole containing compounds useful as angiotensinII antagonists have been disclosed in U.S. Patent 4,880,804 and European Patent Application 392,317. Substituted imidazopyridine containing compounds useful as angiotensin II antagonists have also been disclosed in European Patent Applications 260,613, 399,731 and 412,848 and U.S. Ser. n° 516,286 (filed on April 5, 1990).

Particularly suitable as A II antagonist in the compositions of the present invention, the following drugs can be mentioned : EXP-6803, SC-51316, EXP-7711, L-158809, GR-117289, L-158978, SL-910102, A-81282, FK-739, BMS-180560, CI-996, CGP-48369, LOSARTAN, DUP-532, GR-138950, RWJ46458, KT-3671, BIBR-277, SR-47436, PD-123319, YM-358, SKF-108566, 6SC-50560.

As A II antagonist, Losatran is preferred.

In a preferred embodiment, the combined pharmaceutical composition comprises pharmaceutical carriers suitable for administration to human body.

Indeed, the additive effect in increasing renal blood flow in human is an unexpected result since this is in contrast to animal experiments in which such a benefit of combining the two concerned drugs has not been demonstrated.

In one appropriate embodiment of the composition of the present invention, the dosage forms are adapted for oral administration.

Suitably, both drugs of the compositioni are to be administered 1 to 4 times a day in a method of treatment of human body.

The composition of the present invention may contain 5 to 150 mgs, more particularly 20 to 100 mgs, more particularly 20 to 100 mgs of A II antagonists in a unit dose, in combination with ACE inhibitors, or as single active ingredient in a first dosage form.

The composition of the present invention may contain1 to 100 mgs, more particularly 5 to 50 mgs of ACE inhibitor in a unit dose in association with AII antagonist, or in a second dosage form as a single active ingredient.

In a preferred embodiment of the present invention, the pharmaceutical composition comprises the combination of Enalapril or a pharmaceutically acceptable salt thereof such as maleate salt and Losartan or a pharmaceutically acceptable salt thereof such as potassium salt.

The composition may comprise 20 to 100 mgs of Losartan or a pharmaceutically acceptable salt thereof and 5 to 50 mgs of Enalapril or a pharmaceutically acceptable salt thereof, in the same unit or in two different associated dosage forms of the composition.

More specifically, the pharmaceutical composition comprises 20 mg of Enalapril maleate and 50 mgs of Losartan potassium or a combination of the same drugs in a similar weight ratio.

The pharmaceutical compositions of the present invention can be made by any conventional method, for example known methods of tableting, capsule filling and the like. A simple method is to fill the defined active agents into a capsule; for example, 50 mgs of Losartan potassium and 20 mgs of Enalapril maleate may be filled into a gelatin capsule which is then closed all in conventional manner.

Other characteristics and advantages of the present invention will appear in the following detailed description of one embodiment thereof relating to renal effects of Losartan and Enalapril alone and in combination in healthy volunteers.

Hereafter, the combination of Losartan and Enalapril is shown to have an additive effect in increasing renal blood flow.

Renal haemodynamic and tubular effects of Losartan (angiotensin II antagonist) and Enalapril (ACE) administered alone or in combination were evaluated in 10 healthy subjects in a randomized study.

Mean arterial pressure (MAP), inulin and PAH clearances (GFR and RPF), lithium (Cl_{Li}), sodium (Cl_{Na}), and uric acid (Cl_{AU}) clearances were assessed after placebo (P), Losartan (50 mg as a single oral dose) (L), Enalapril (20 mg) (E) or Enalapril + Losartan (E+L) administration. Lithium clearance was used to determine fractionnal proximal and distal sodium reabsorption (FDR_{Na}). Values were obtained 90 minutes after drug

| | P | L | E | E+L |
|---|---|---|---|---|
| GFR (ml/min.1.73 m²) | 110±6 | 116±5 | 111±3 | 114±3 |
| RPF (ml/min.1.73 m²) | 792±39 | 890±47* | 871±49 | 931±44*^{Δ} |
| MAP (mmHg) | 83±2 | 82±3 | 78±3* | 77±2* |
| Cl_{Na} (ml/min.1.73 m²) | 1.7±0.3 | 3±0.4* | 2±0.2 | 2.7±0.3*^{Δ} |
| Cl_{Li} (ml/min.1.73 m²) | 36±2 | 34±2 | 34±1 | 30±2*^{Δ} |
| FDR_{Na} | 0.95±0.01 | 0.91±0.01* | 0.94±0.01 | 0.91±0.01*^{Δ} |
| Cl_{AU} (ml/min.1.73 m²) | 9.6±0.9 | 15.7±1.6* | 10.4±0.5 | 16.4±1.9*^{Δ} |

| | | | | |
|---|---|---|---|---|
| *p<0.5 vs placebo (P) ; | | | | |
| ^{Δ}p<0.05 vs Enalapril alone (E) | | | | |

Acute Losartan administration did not alter neither glomerular filtration rate nor blood pressure. Losartan increased renal plasma flow, sodium and uric acid clearances. This enhanced natriuresis resulted from a post-proximal fall in sodium reabsorption. Enalapril slightly decreased blood pressure. Losartan did not potentiate this Enalapril effect on blood pressure while the two molecules have an additive effect on renal blood flow rise.

## Claims

1. A combined pharmaceutical composition comprising a combination of at least one angiotensin converting enzyme (ACE) inhibitor and at least one AII antagonist (Angiotensin II antagonist) for simultaneous, separate or sequential administration.

2. A pharmaceutical composition as claimed in claim 1, wherein the angiotensin converting enzyme inhibitor is in first dosage form and the AII antagonist is in a second dosage form.

3. A pharmaceutical composition as claimed in any one of claims 1 or 2, wherein the angiotensin converting enzyme inhibitor and the AII antagonist are in a single unit dosage.

4. A pharmaceutical composition to enhance renal blood flow according to claim 1.

5. Pharmaceutical composition according to any one of claims 1 to 4 comprising pharmaceutical carriers suitable for administration to human body.

6. Pharmaceutical composition according to any one of claims 1 to 5 for treating or preventing cardiovascular diseases.

7. A combined composition as claimed in any of claims 1 to 6, wherein both medicaments are to be administered from 1 to 4 times a day.

8. Pharmaceutical composition according to any one of claims 1 to 7, wherein the ACE inhibitors are selected from the group consisting of enalapril, lisinopril, ceranapril, imidapril, captopril, DU-1777, zabicipril, utibapril, AB-47, cilazapril, zofenopril, fosinopril, delapril, spirapril, perindopril, libenzapril, moexipril, MDL-100240, quinapril, trandolapril, benazepril, quinaprilat, FPL-66564, Synecor, Prentyl, BIBS39, temocapril, idrapril, ramipril.

9. Pharmaceutical composition according to any one of claims 1 to 8, wherein the AII antagonists are selected from the group consisting of EXP-6803, SC-51316, EXP-7711, L-158809, GR-117289, L-158978, SL-910102, A-81282, FK-739, BMS-180560, CI-996, CGP-48369, LOSARTAN, DUP-532, GR-138950, RWJ46458, KT-3671, BIBR-277, SR-47436, PD-123319, YM-358, SKF-108566, 6SC-50560.

10. A pharmaceutical composition as claimed in any one claims 1 to 9 in a dosage form adapted for oral administration.

11. A pharmaceutical composition as claimed in any one of claims 1 to 10 which contains in a unit dose 5 to 150 mgs of an angiotensin II antagonist.

12. A pharmaceutical composition as claimed in claim 11 which contains in a unit dose 20 to 100 mgs of an A II antagonist.

13. A pharmaceutical composition as claimed in any one of claims 1 to 12, wherein the A II antagonist is Losartan or a pharmaceutically acceptable salt thereof such as the potassium salt.

14. A pharmaceutical composition as claimed in any one of claims 1 to 13 which contains in a unit dose 1 to 100 mgs of ACE inhibitor.

15. A pharmaceutical composition as claimed in claim 14 which contains in a unit dose 5 to 50 mgs of ACE inhibitor.

16. A pharmaceutical composition as claimed in any one of claims 1 to 14, wherein the ACE inhibitor is Enalapril, Lisinopril, Captopril, Ramipril, Cilazapril, Quinapril or a pharmaceutically acceptable salt thereof.

17. A pharmaceutical composition as claimed in claim16, wherein the ACE inhibitor is Enalapril maleate.

18. A pharmaceutical composition according to any one of claims 1 to 17 which comprises 20 to 100 mgs of Losartan or a pharmaceutically acceptable salt thereof and 5 to 50 mgs of Enalapril or a pharmaceutically acceptable salt thereof, in the same unit dose or in two different associated dosage forms of the composition.

19. A pharmaceutical composition as claimed in claim 18 which comprises 50 mgs of Losartan and 20 mgs of Enalapril maleate.
